# EUROPEAN PATENT APPLICATION

(11) **EP 3 808 359 A1**
(43) Date of publication of application: **21.04.2021**
(21) Application number: 20020470.9
(22) Date of filing: 13.10.2020
(51) Int. Cl.: A61K 36/324, A61P 17/00

(54) **COMBINATION OF NATURAL ACTIVE INGREDIENTS PROMOTING APOPTOSIS IN DAMAGED OR SUFFERING SKIN TISSUES**

(30) Priority: 14.10.2019 IT 201900018659
(71) Applicant: Valetudo S.r.l. Con Unico Socio, 24030 Presezzo (BG) (IT)
(72) Inventor: Bortolin, Vittorino, 24030 Presezzo (BG) (IT)
(74) Representative: Lecce, Giovanni

(57) **Abstract**

A combination of natural active ingredients that promote apoptosis, i.e. programmed cellular death in damaged or suffering skin tissues, consisting of compositions comprising resin extracts of Boswellia Serrata or its pure active ingredients of the family of Boswellic acids and phytosterols or phytostanols or derivatives thereof.

## Description

The present invention relates to a combination of natural active ingredients promoting apoptosis, that is the planned cell death in damaged or suffering skin tissues.

In particular the present invention relates to a combination of natural active ingredients of which the ability to promote the final phase of the apoptosis process has been discovered, with other active ingredients with the ability to promote the initial phase of the same process, thus allowing a synergistic effect to be obtained, and therefore a greater pro-apoptotic effectiveness, useful in the treatment of certain dermatological disorders.

More specifically, the present invention refers to a combination of resin extracts of Boswellia Serrata or its pure active ingredients of the family of Boswellic acids, which act in the initial phase of the apoptosis process, with natural substances of the family of phytosterols/phytostanols or their derivatives (esters and/or glucosides of phytosterols or phytostanols) which have the capacity to selectively favour the final phase of the apoptosis process (according to a mechanism of action discovered by the inventor) thereby allowing important advantages to be obtained in terms of overall pro-apoptotic efficacy.

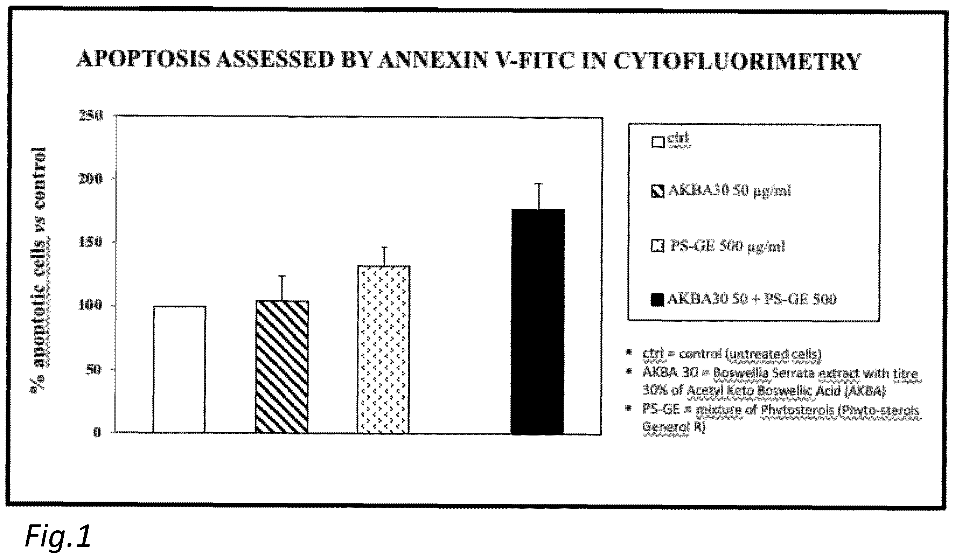

In particular, this combination of natural substances allows an important synergistic effect (booster effect) to be obtained, which can be demonstrated "in vitro", as shown in Figure 1 below, and which can be used advantageously in the treatment of some specific dermatological disorders.

It is known that the cells that make up every tissue in our body are subject to continuous renewal. It is estimated that 50 to 70 billion new cells are formed every day in a healthy adult human body.

In order to maintain the physiological balance of the body, as many cells must die. The old or damaged or imperfect cells of our body are, in fact, subjected to a continuous process of elimination through a perfect mechanism of programmed, clean and rapid death, called apoptosis.

Apoptosis is an extremely useful process because it protects the body from cells which are defective, damaged by radiation or infected by viruses, inducing "suicide" once discovered.

Apoptosis is also one of the most important defences against cancer. Sometimes cancer cells become malignant precisely because they disable their apoptosis mechanism.

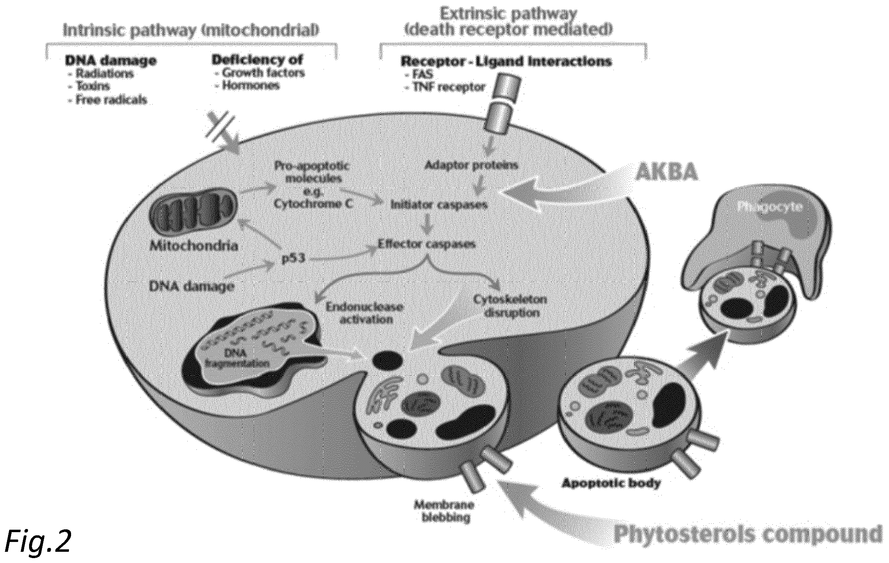

It is known that the process of apoptosis consists of two distinct phases:
- step 1: the start of the process which takes place through the activation of the caspases;
- step 2: the conclusion of the process that takes place thanks to the emission of a phagocyte beckoning signal (see Figure 2 below).

Caspases are the enzymes responsible for demolishing the cell from the inside, their activation occurs as a result of signals of different types that all indicate the need for a certain cell to be eliminated by apoptosis.

Activation of the caspases is enabled by the triggering of a cascade of molecular events that can be induced in various ways, along two alternative paths, one known as the intrinsic (or mitochondrial) path and the other as the extrinsic (or death signals) path.

Caspases, regardless of the path that led to their activation, demolish the cell structure and lead the cell to collapse. As a result, the cell membrane that delimits the cell, which is made up of a double layer of phospholipids and cholesterol, also goes into crisis, losing its compactness. This loss of compactness is essential for the triggering of step 2 of the process: the phagocytation (phagocytosis) of the dying cell.

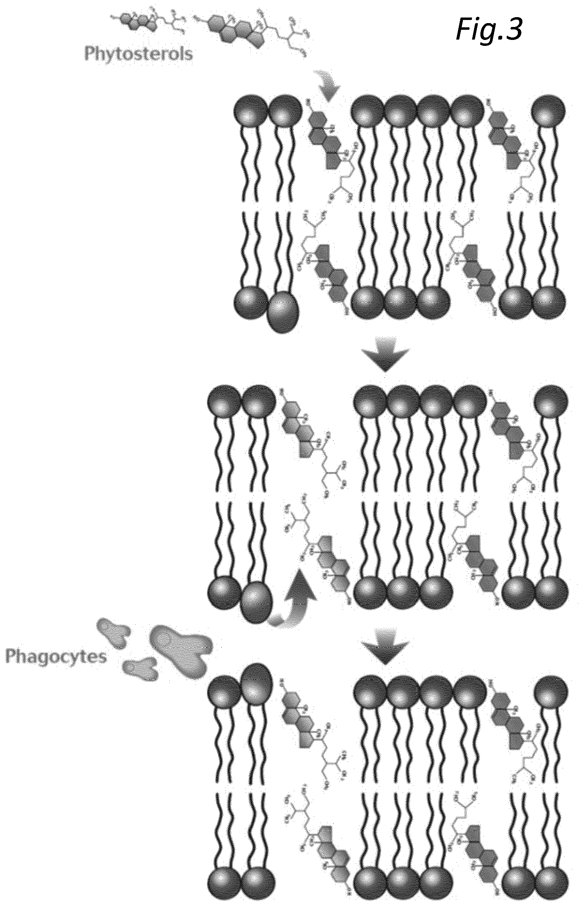

The final phase of apoptosis in fact requires the intervention of phagocytes, specialized cells that are required to incorporate and "digest" the dying cell. The task of beckoning the phagocytes is entrusted to one of the phospholipidic molecules making up the cell membrane, phosphatidylserine, which is stimulated to free itself from its fixed position inside the cell membrane, moving outwards. This "externalization" movement of the phosphatidylserine is the indispensable beckoning signal for the phagocytes (see also Figure 3 below). The intervention of phagocytes is a crucial event for the successful outcome of the apoptotic process, as it prevents the content of the dying cell from being poured into the surrounding space, with a consequent inflammatory reaction.

In recent years, scientific research has been working hard to find ways to help damaged or suffering cells to take the virtuous path of death by apoptosis.

Up to now, the path that has been followed to achieve the objective has always been to facilitate the start of the process (step 1).

The studies that led to the present invention set out to verify:
1) if active ingredients capable of promoting the final phase of the apoptosis process (step 2) exist
2) if combinations between ingredients acting in the apoptosis starting phase (step 1) with ingredients capable of acting in the final phase of the same process (step 2) allow for greater pro-apoptotic effectiveness.

Among the substances with initiating activity of the process, resin extracts of Boswellia Serrata and its active ingredients of the family of Boswellic acids have been taken into consideration.

The active ingredients of the family of Boswellic Acids are typically taken to mean the compounds chosen in the group including Acetyl Keto-Boswellic Acid (AKBA), Boswellic Acetyl Acid, Boswellic Keto Acid, Boswellic Acid, both as alpha and beta isomers, either alone or mixed together, preferably the active ingredient Acetyl Keto-Boswellic Acid (AKBA).

In fact, there are numerous "in vitro" studies that recognize various different therapeutic virtues of the extracts of Boswellia Serrata and its active ingredients of the Boswellic acid family, including the ability to induce apoptosis through activation of the caspases.

Despite the good results of the "in vitro" studies carried out so far, the practical use of these active ingredients in the dermatological field as anti-tumour/cytostatic/anti-proliferative/anti-inflammatory adjuvants has not been very successful, as their pro-apoptotic efficacy "in vivo" proved to be less than expected.

This invention is based on the discovery that
1. natural substances of the phytosterols/phytostanols family and their derivatives have the ability to facilitate the final stage of the apoptosis process;
2. natural substances of the phytosterols/phytostanols family and their derivatives have the ability to decisively increase the pro-apoptotic efficacy of Boswellia Serrata resin extracts or Boswellic acids (booster effect).

Phytosterols and phytostanols are substances present in many plants, from which they are obtained, widely known and used in dietary supplements for their ability to act as substitutes and antagonists of cholesterol, of which they hinder the intestinal absorption and damage induced to the arteries by hypercholesterolemia.

Phytosterols are also commonly used in dermatology as cholesterol substitutes, with the specific aim of strengthening, as is well known, the skin barrier of the epidermis, thus protecting the skin from dehydration and contact with external allergenic or irritating substances. In this application the phytosterols act by becoming part of the so-called "intercorneocyte cement", a mixture of substances originating from cell disintegration which contributes to welding together the dead cells (the corneocytes) of the horny layer of the epidermis, making it more compact and impermeable to external agents and preserving the skin from dehydration.

The studies underlying this invention have shown that phytosterols and/or phytostanols or their derivatives (phytosterol esters and/or glucosides of phytosterols or phytostanols) may perform another action of dermatological interest, completely different from the above and never described before. This action no longer concerns the dead cells of the stratum corneum but the living cells of the deeper layers and consists in their ability (of phytosterols and/or phytostanols or their derivatives) to modify the conformation of the cell membranes of the skin cells that are candidates for apoptosis, thus favouring the so-called "phosphatidylyserine externalization", a biochemical event considered crucial for apoptosis to be concluded with the essential final act, the phagocytosis of the dying cell (as shown in Figure 2 below). As known, for phagocytosis to take place a specific message from the dying cells must reach the phagocytes. This message consists of a change of position of some specific components (phosphatidylserine molecules) of the cell membranes. This change of position is favoured by the presence of phytosterols and/or phytostanols and/or their derivatives (as shown in Figure 3 below).

### Brief description of the figures

Figure 1 describes an "in vitro" test *(Simona Serini and Gabriella Calviello* - *Institute of General Pathology -Faculty of Medicine and Surgery - Fondazione Policlinico A. Gemelli IRCCS- Università Cattolica del Sacro Cuore* - *L. go F. Vito, 1* - *00168 Rome, Italy)* showing the booster effect (synergistic effect) of phytosterols on apoptosis induced by a resin extract of Boswellia Serrata with a 30% titre in AKBA (Acetyl Keto Boswellic Acid).
Figure 2 shows the two phases of apoptosis:
   - the initial phase, which may be facilitated by the use of Boswellia Serrata extracts, AKBA or other Boswellic acids,
   - the final phase of apoptosis, which ends with the intervention of the phagocytes, specialized cells that incorporate and "digest" the dying cell. The beckoning of the phagocytes is based on a "signal" which is amplified by the presence of substances, such as phytosterols, which modify the structure of the membrane of the dying cell.
   AKBA means, as mentioned above, Acetyl Keto-Boswellic Acid while FAS is a cell membrane receptor which, when activated, triggers cell apoptosis.
Figure 3 describes the behaviour of phytosterols towards cell membranes, according to the mechanism of action discovered by the inventor. This figure shows how phytosterols, taking advantage of their similarity with cholesterol, displace and replace it, thus making cell membranes more mobile and facilitating the phosphatidylserine externalization process. This outward displacement of phosphatidylserine is the beckoning signal for the phagocytes which promptly intervene to incorporate and "digest" the cell (this event is in fact known as "eat-me" signal) thus allowing a rapid conclusion of the apoptosis process.

The aim of the invention is to propose a combination of natural active ingredients promoting apoptosis in damaged or suffering skin tissues.

More specifically, the aim of the invention is to propose a combination of natural active ingredients that act in the initial phase of apoptosis with ingredients capable of acting in the final phase of the same process, allowing for greater pro-apoptotic efficacy.

This purpose is achieved by combining natural substances of the family of phytosterols/phytostanols or their derivatives (esters and/or glucosides of phytosterols or phytostanols), which are able to favour the final phase of the apoptosis process (step 2), with resin extracts of Boswellia Serrata or its pure active ingredients of the family of Boswellic acids, effective in the start-up phase (step 1), thus achieving important advantages in terms of overall pro-apoptotic efficacy.

A further aim of the present invention is to propose a combination of natural active ingredients that promote apoptosis in skin diseases. Among the indications which this invention aims to treat are:
- skin diseases of auto-immune origin such as psoriasis;
- pre-cancerous forms such as actinic keratosis;
- damage to skin cells caused by aggressive medical treatments, both physical (radiotherapy) and chemical-pharmacological;
- damage caused to skin cells by accidental factors such as burns, sunburn, contact with caustic chemicals, injury or abrasion;
- allergic or contact inflammatory dermatitis;
- atopic dermatitis;
- Herpes Virus skin disease;
- acne vulgaris;
- rosacea.

A further purpose of this invention is to propose a combination of natural active ingredients that promote apoptosis in damaged or suffering skin tissues which comprises pharmaceutical preparations, medical devices or cosmetics.

The purpose of this invention is to provide a combination of natural active ingredients, which promote apoptosis in damaged or suffering skin tissues, in compositions intended for topical use, both pharmaceutical and cosmetic. These compositions can take the form of emulsions (lotion or cream or ointment), or solutions, lotions or gels, or even shampoos, sprays or pressurised foams with propellant gases.

A further purpose of the present invention is to provide emulsions that can comprise a wide range of optional components, to be added to the preparation after the oily and aqueous phase have been combined or mixed together, such as fragrances, plant extracts, sunscreens, colouring agents.

A no less important purpose of this invention is to provide a combination of natural active ingredients that promote apoptosis in damaged or suffering skin tissues in the form of compositions, the components of which are commercially available or can be easily prepared following procedures known in the art.

These and other purposes are achieved by combining natural substances of the family of phytosterols or phytostanols or their derivatives with resin extracts of Boswellia Serrata or with Boswellic acids according to this invention.

This invention will now be described by way of non-limiting example, according to some preferred embodiments.

Preferably the compositions referred to in this invention include resin extracts of Boswellia Serrata or its pure active ingredients of the family of Boswellic acids and phytosterols, in particular beta-sitosterol, campesterol, stigmasterol, brassicasterol, and/or phytosterols and/or phytostanols and/or phytostanol esters or glucosides, either alone or as mixtures of plant origin that significantly contain, incorporated in a physiologically acceptable excipient, i.e. compatible with the skin or scalp, non-toxic, nonreactive.

The term "significantly contain' means a content of phytosterols and phytostanols or their derivatives in the mixture of plant origin of at least 5 % by total weight of such mixture.

More preferably, the compositions according to this invention comprise Boswellia Serrata resin extracts and phytosterols or phytostanols or their derivatives (esters or glucosides of phytosterols and/or phytostanols) in a ratio of 10:1 to 1: 5.

Even more preferably the compositions comprise Boswellia Serrata resin extracts and phytosterols from rapeseed or soya or avocado or olive oils and a physiologically acceptable excipient and can typically be in the form of oil emulsion in water (O/A) or water in oil (A/O), including an oily phase and an aqueous phase, or in the form of gel, spray, shampoo, solution for tissues for example or water-based or hydro-alcoholic lotion or foam under pressure with propellant gases.

In the oil-in-water or water-in-oil emulsions the oily phase may contain branched and non-branched hydrocarbons such as vaseline oil, paraffin, squalane, polyisobutene, polydecenes, possibly hydrogenated, preferably vaseline oil and hydrogenated polydecene; silicone oils, e.g. dimethicone; fatty acids selected from saturated and partially unsaturated fatty acids having 8 to 20 carbon atoms, e.g. lauric, myristic, palmitic, stearic, isostearic, oleic, linoleic, eicosanoic, docosanoic, erucic; C1-C20 alkyl and alkenyl esters of saturated and partially unsaturated fatty acids having 10 to 30 carbon atoms, such as decyl oleate, isodecyl oleate, dioctil maleate, diisopropyl palmitate, isohexyl palmitate, ethylhexyl palmitate, lauryl lactate, myristil lactate, cetyl lactate, isostearyl neopentanate, ethylhexyl isostearate, myristil myristate, hexyl laurate, cetyl palmitate, isopropril palmitate, hexadecyl stearate, decyl stearate, isopropyl isostearate, dibutyl adipate, diisopropyl adipate, diisohexyl adipate, dihexyldecyl adipate, isocetyl stearoyl stearate, C12-C15 alkyl lactate, cetearyl isononanoate and mixtures thereof, preferably ethylhexyl palmitate, decyl oleate, isopropyl myristate, isopropyl palmitate, myristil myristate, isostearyl neopentanoate, cetearyl isononanoate, dibutyl adipate, C12-C15 alkyl lactate.

The oily phase may also contain cetyl alcohol, stearyl alcohol, glyceryl stearate, polysorbates, esters or ethers of C12 to C18 fatty acids with polyethylene glycol.

The oily phase may also contain alkyl-phthalimides, for example butyl-phthalimide or isopropyl-phthalimide.

Components of the aqueous phase may include water, alcohols such as denatured ethyl alcohol or isopropyl alcohol, organic salts such as sodium citrate, sodium potassium tartrate, potassium sorbate, sodium benzoate and ethylenediaminetetracetic acid (EDTA); inorganic salts such as sodium chloride, sodium metabisulphite, magnesium sulphate; buffering agents such as sodium hydroxide, sodium bicarbonate, sodium hydrogen phosphate and mixtures thereof; solubilising agents such as dimethyl isosorbide; organic acids such as salicylic or citric acid; preservatives such as parabens such as methylparaben, phenoxyethanol, chlorhexidine, chlorfenesin, imidazolidinyl urea, glycine derivatives; essential oils such as eucalyptus, thyme, cinnamon, geranium; amino acids such as arginine, betaine and lysine, and their derivatives; gelling agents, e.g. cellulose derivatives, alginates, carrageenans, guar gum and xanthan gum derivatives, acrylic acid polymers such as polyacrylates, methacrylates, carbomers; vitamins such as niacinamide.

The aqueous phase may also include urea, glycerol, propylene glycol, butylene glycol, ethyl alcohol, isopropyl alcohol, polyalcohols, aminoalcohols such as triethanolamine, saccharide components derived from starch or cellulose.

Preferably the water used in this invention is deionised water. The content of water in the preparation of this invention is relative to the total content of the other components used, such that the total weight of the preparation is equal to 100% by weight of the topical preparation.

The resin extracts of Boswellia Serrata or its active ingredients of the family of Boswellic acids are present in the various formulations in quantities between 0.05 and 12% by weight, more preferably between 0.1 and 8% by weight of the preparation.

Phytosterols or phytostanols, or derivatives thereof, or natural mixtures containing them in significant amounts, are present in the different formulations in quantities between 0.05 and 10% by weight, more preferably between 0.1 and 5% by weight.

The emulsions (lotions, creams, ointments) of the present invention may include, if desired, a wide range of optional components, such as fragrances, plant extracts, sun filters, colouring agents. These optional components can be added to the preparation after the oily and aqueous phase have been combined or mixed together.

The method of preparation of compositions for topical application, on the surface of mammalian skin, in particular human skin, for cosmetic and/or pharmaceutical use referred to in this invention includes the mixing of active ingredients with a pro-apoptotic action with a physiologically acceptable carrier.

The emulsions referred to in this invention (typically lotions, creams or ointments) can be prepared by any method known in the art. In general, such methods include the step of preparing the oily phase and the aqueous phase in separate containers and then combining the two phases.

The oily phase is obtained by mixing its components by constant stirring in a mixing container separate from the mixing container containing the components of the aqueous phase, for a time that depends on the size of the mixing container used, and thus the appropriate mixing time may vary from a few minutes to hours. If required, the oily phase can be heated to a temperature of approximately 40° to 90 °C, preferably from 65° to approximately 80 °C, to allow the solid components to dissolve.

The aqueous phase is obtained by dissolving solid and liquid components in water under gentle stirring, for a time that depends on the size of the mixing container used and thus the appropriate mixing time may vary from a few minutes to hours.

If necessary, to obtain the emulsion, the aqueous phase can be heated to the same melting temperature as the oily phase before mixing the two phases.

The oily phase is then mixed together with the watery phase under constant stirring until it becomes homogeneous. The mixing of the two phases can take place in the same container or tank in which the aqueous or oily phase was originally mixed.

The resulting mixture is cooled, for example, by circulation of cold water in the cavity of the vessel in which the preparation is carried out until the mixture reaches the desired temperature. If required, a component as defined above may be added to the mixture during or after the cooling phase under continuous slow mixing. At the end of the process the mixture can be stored in a storage tank and then packaged in the final containers.

The gels referred to in this invention may consist of various components, some of which are common to emulsions and mentioned above or indicated in the examples below.

The gels referred to in this invention can be prepared by any method known in the art. In general, these methods involve the preparation of an aqueous phase which is then gelled. The aqueous phase is obtained by dissolving solid and liquid components in water under stirring, for a time that depends on the intensity of said stirring and on the size of the mixing container used and thus the appropriate mixing time may vary from a few minutes to hours. The aqueous phase is gelled with a gelling agent that can be added to the aqueous phase during or at the end of the stirring process. The preparation also includes the control and correction of the final pH of the product.

The lotions, sprays, shampoos and solutions referred to in this invention may consist of various components, some of which are common to emulsions and mentioned above or indicated in the examples below.

Said formulations can be prepared by any method known in the art. These methods involve the preparation of an aqueous phase which is, as required, suitably scented, coloured and thickened. The aqueous phase is obtained by dissolving solid and liquid components in water under stirring, for a time that depends on the intensity of said stirring and on the size of the mixing container used and thus the appropriate mixing time can vary from a few minutes to hours. The aqueous phase can be perfumed, coloured and/or thickened with perfumes, dyes or thickeners that can be added to the aqueous phase during or at the end of the stirring process. The preparation also includes the control and correction of the final pH of the product.

In the range of formulations and compositions referred to in this invention, various ingredients for cosmetic and/or pharmaceutical use may be used.

Both pharmaceutical and cosmetic preparations can be in the form of emulsion, shampoo, solution, lotion or skin spray. In these cases, all ingredients recognized as suitable for pharmaceutical and/or cosmetic products may be introduced into the physiologically acceptable carrier and used to prepare emulsions, shampoos, solutions, lotions, gels or skin sprays within the scope of this invention.

In order to better describe the present invention, 7 examples are given, which describe only some of the possible compositions of the invention. Although such examples use selected formulations in accordance with this invention, it is clear that such examples are illustrative only and not limiting. All the phytosterols and/or phytostanols or natural mixtures and/or esters thereof may be used in accordance with the indications of this invention, in formulations similar to those given below.

All the components of the compositions of this invention are commercially available or can be easily prepared following procedures known in the art.

The ingredient "Aqua" mentioned in the examples below is always deionised water.

All parts, percentages and proportions referred to and indicated in the claims are understood to be the total weight of the composition, unless otherwise stated.

The names of the ingredients used and shown in the examples follow the International Nomenclature of Cosmetic Ingredients (INCI) and are those officially recognized in the cosmetic sector.

### EXAMPLE 1: Sunburn cream (emulsion without emulsifier) - the percentages are here expressed as % by weight (p/w)

Part A: Hydrogenated Polydecene 6.00 % Prunus Amygdalus Dulcis Oil 6.00 % Isopropyl Palmitate 3.00 % Brassica Campestris Sterols 0.50 %;
Part B: Ammonium Acryloyldimethyltaurate/VP Copolymer 1.00 %;
Part C: Aqua 65.20 % Disodium EDTA 0.10 % Glycerin 4.00 %;
Part D: Parfum 0.20 %, Boswellia Serrata Resin Extract 1.50 % Alcohol denat. 10.50 % Dimethyl Isosorbide 2.00 %.

### Preparation:

I) Heat Part A to about 80°C under stirring until completely homogeneous;
II) Add slowly and under constant stirring Part B and then Part C to the mixture referred to in step I) and then add Part D;
III) Homogenise the emulsion thus obtained.

### EXAMPLE 2: Adjuvant face cream for actinic keratosis - the percentages are here expressed as % by weight (p/w)

Part A: Ceteareth-25 2.00 % Ceteareth-5 3.00 % Glyceryl Stearate 2.00 % Cetyl Alcohol 3.00 % Caprylic/Capric Triglyceride 7.00 % Phytosteryl Oleate 1.20 % Dimethicone 0.50 % Isopropyl Palmitate 5.00 % Tocopheryl Acetate 0.50 %;
Part B: Aqua 63,00 % Disodium EDTA 0.1 % Carbomer 0.20 %;
Part C: Sodium Hydroxide (10 % in water) 0.80 % Phenoxyethanol 0.50 % Methylparaben 0.20 % Glycerin 5.00 %;
Part D: Boswellia Serrata Resin Extract 2.00 % Alcohol denat. 4.00 %.

### Preparation:

(I) Melt Part A at approximately 80° C;
II) Heat Part B to approximately 80°C under stirring;
(III) Add the mixture obtained in step (II) to that obtained in step (I) and cool under stirring to room temperature;
(IV) Add Part C to that obtained in step III);
(V) Add Part D to that obtained in step IV);
VI) Homogenise the emulsion.

### EXAMPLE 3: Solution for soaked tissues to treat skin lesions caused by caustic or toxic chemicals - the percentages are here expressed as % by weight (p/w)

Part A: PEG-7 Glyceryl Cocoate 3.00 % PEG-40 Hydrogenated Castor Oil 2.20 % Polysorbate-20 2.00 % Persea Gratissima Sterols 0.20 %;
Part B: Aqua 67.50 % Glycerin 7.50 % Alcohol denat.16.00 % Boswellia Serrata Resin Extract 0.60 % Citric Acid (10 % in water) 0.90 % Disodium EDTA 0.10 %.

### Preparation:

(I) Heat part A to 85 °C under stirring until clear and then cool to room temperature.
(II) Add Part A to Part B at room temperature and mix until a clear solution is obtained.

### EXAMPLE 4: Hair cream, adjuvant in the treatment of psoriasis of the scalp - the percentages are here expressed as % by weight (p/w)

Part A: Behentrimonium Chloride 2.00 % Ceteareth-25 1.50 % Cetearyl alcohol 2.00 % Paraffinum liquidum 2.00 % Boswellia Serrata Resin Extract 1.00 % Beta-sitosterol 0.20 % Beta-sitosteryl acetate 0.2 %;
Part B: Aqua 85.80 %; Part C Glycerin 5.00 % Parfum 0.30 %;
Part D: Citric Acid as needed to obtain a pH between 6.00 and 6.50.

### Preparation:

I) Melt Part A at 80°C;
II) Heat Part B to 80°C;
(III) Add what is obtained in II) to what is obtained in I) under stirring;
(IV) Cool the mixture referred to in step III) under stirring;
V) At 35°C add the components of Part C to what is obtained in IV);
VI) Adjust the pH with Part D to values between 6.00 and 6.50.

### EXAMPLE 5: Adjuvant body cream for psoriasis - percentages are here expressed as % by weight (p/w)

Part A: PEG-100 Stearate 2.5 % Glyceryl Stearate 4.00 % Cetyl Alcohol 3.00 % Paraffinum Liquidum 7.00 % Macadamia Ternifolia Seed Oil 7.00 % Brassica Campestris Sterols 0.60 % Stigmastanol Maltoside 0.40 % Dimethicone 0.50 % Isopropyl Palmitate 3.00 % Potassium Cetyl Phosphate 0.5 % Tocopheryl Acetate 0.50 %;
Part B: Aqua 54.00 % Disodium EDTA 0.10 % Carbomer 0.20 %; Part C: Sodium Hydroxide (10 % in water) 0.80 % Phenoxyethanol 0.50 % Methylparaben 0.20 % Glycerin 4.00 % Urea 4 %; Part D Boswellia Serrata Resin Extract 3.00 % Alcohol denat. 4.20 %.

### Preparation:

(I) Melt Part A at approximately 80° C;
II) Heat Part B to approximately 80°C under stirring;
(III) Add the mixture obtained in step (I) to that obtained in step (II) and cool under stirring to room temperature;
IV) Add Part C to what is obtained in step III);
(V) Add Part D to that obtained in step IV);
VI) Homogenise the emulsion.

### EXAMPLE 6: Sprayable solution as an adjuvant in the treatment of herpes zoster - the percentages are here expressed as % by weight (p/w)

Part A: Butylphthalimide and Isopropylphthalimide 7.30 % Brassica Campestris Sterols 0.20 % C12-C15 Alkyl Lactate 17.50 % Dibutyl Adipate 7.00 % Polysorbate 20 1.00 %;
Part B: Alcohol Denat. 60.00 % Boswellia Serrata Resin Extract 4.50 % Glycerin 2.5 %.

### Preparation:

I) Heat Part A to about 85° C under constant stirring until clear and then leave to cool to room temperature under stirring;
(II) Prepare Part B by stirring at room temperature until clear;
(III) Add what is obtained in step (I) to what is obtained in step (II) and continue stirring.

### EXAMPLE 7: Fluid cream as an adjuvant in the healing of wounds, abrasions or skin burns - the percentages are here expressed as % by weight (p/w)

Part A: Butyl phthalimide and Isopropyl phthalimide 7.00 % Glyceryl Stearate 4.00 % Cetyl Alcohol 3.00 % Potassium Cetyl Phosphate 2.00 % Brassica Campestris Sterols 1.00 % Stearic Acid 2.00 Sodium Stearoyl Lactylate 1.50 % Dimethicone 0.50 % Isopropyl Palmitate 3.00 % Tocopheryl Acetate 2.50 %;
Part B: Aqua 55.80% Disodium EDTA 0.10% Xanthan Gum 0.20% Propylene Glycol 2.5% Part C: Alcohol Denat. 5.00 % Boswellia Serrata Resin Extract 3.50 % Part D: Phenoxyethanol 0.70 % Benzyl Alcohol 0.30 % Ethylhexylglycerin 0.10 % Pentylene Glycol 3.5 % Panthenol 1.50 %, Allantoin 0.30 %.

### Preparation:

(I) Melt Part A at approximately 85° C;
II) Heat Part B to approximately 85°C under stirring;
(III) Add the mixture obtained in step (I) to that obtained in step (II) and cool under stirring to room temperature;
IV) Add Part C to what is obtained in step III) and continue stirring;
V) Add Part D to what is obtained in step IV) and continue stirring;
VI) Homogenise the emulsion.

Despite the invention having been described with reference to some of its embodiments, given solely by way of a non-limiting example, numerous modifications and variants to the formulations of the present invention will appear evident to a person skilled in the art in the light of the above description. The present invention therefore sets out to embrace all the modifications and variants which fall within the scope of protection of the following claims.

## Claims

1. A combination of natural active ingredients that promote apoptosis in damaged or suffering skin tissues, consisting of compositions **characterized in that** they comprise resin extracts of Boswellia Serrata or its pure active ingredients of the family of Boswellic acids and phytosterols or phytostanols or derivatives thereof selected among beta-sitosterol, campesterol, stigmasterol, brassicasterol, and/or esters or glucosides of phytosterols and/or phytostanols and/or esters or glucosides of phytostanols, either alone or as mixtures of plant origin that significantly containing them, incorporated in a physiologically acceptable excipient.

2. The compositions according to claim 1, **characterized in that** they comprise resin extracts of Boswellia Serrata and phytosterols or phytostanols or derivatives thereof in a ratio of 10:1 to 1: 5.

3. The compositions according to claim 1, **characterized in that** they comprise resin extracts of Boswellia Serrata and phytosterols from rapeseed or soybean or avocado or olive oils and a physiologically acceptable excipient.

4. The compositions according to claim 1, **characterized in that** the resin extracts of Boswellia Serrata or its active ingredients of the Boswellic acid family are present in the various formulations in amounts ranging from 0.05 to 12 % by weight.

5. The compositions according to claim 1, **characterized in that** the phytosterols or phytostanols or derivatives thereof, or natural mixtures containing them in significant amounts, are present in the various formulations in amounts ranging from 0.05 to 10% by weight.

6. the compositions according to claim 1, **characterized in that** they comprise pharmaceutical preparations, medical devices and cosmetics suitable for promoting apoptosis in the context of skin diseases of auto-immune origin and of pre-cancerous origin, of skin damage caused by aggressive medical treatments and by accidental factors, of inflammatory allergic dermatitis and of chronic inflammatory dermatitis, of Herpes Virus skin diseases, of acne vulgaris and of rosacea.

7. The compositions according to claim 1, **characterized in that** they are in the form of an oil-in- water or water-in -oil emulsion, comprising an oily phase and an aqueous phase, in the form of gel, spray, shampoo, solutions, aqueous or hydroalcoholic lotions or foam under pressure with propellant gases.

8. The emulsions according to claim 7, **characterized in that** the oily phase contains branched and unbranched hydrocarbons, silicone oils, fatty acids selected among saturated and partially unsaturated fatty acids having 8 to 20 carbon atoms, C1-C20 alkyl and alkenyl esters of saturated and partially unsaturated fatty acids having 10 to 30 carbon atoms and mixtures thereof.

9. The emulsions according to claim 7, **characterized in that** the oily phase also contains cetyl alcohol, stearyl alcohol, glyceryl stearate, polysorbates, esters or ethers of C12 to C18 fatty acids with polyethylene glycol.

10. The emulsions according to claim 7, **characterised in that** the oily phase also contains alkyl phthalimides.

11. The emulsions according to claim 7, **characterized in that** the components of the aqueous phase include water, alcohols, organic salts, inorganic salts, buffering agents and mixtures thereof, solubilizing agents, organic acids, preservatives, essential oils, amino acids and derivatives thereof, gelling agents, vitamins.

12. The emulsions according to claim 7, **characterized in that** the aqueous phase also comprises urea, glycerol, propylene glycol, butylene glycol, ethyl alcohol, isopropyl alcohol, polyalcohols, amino alcohols, saccharide components derived from starch or cellulose.

13. The emulsions according to claim 7, **characterized in that** the water used is deionized water, the content of which in the preparation is in respect to the total content of the other components used in such a way that the total weight of the preparation is equal to 100% by weight of the topical preparation.

14. The emulsions according to claim 7, **characterized in that** they comprise optional components such as fragrances, plant extracts, sunscreens, colouring agents, to be added to the preparation after the oily and aqueous phase have been combined or mixed together.

15. Compositions in the form of gel, spray, shampoo, solutions, lotions or foam under pressure according to claim 7, **characterized in that** they comprise different components common to the emulsions.

16. The compositions according to claim 7, **characterized in that** they comprise ingredients recognized as suitable for pharmaceutical and/or cosmetic products introduced into the physiologically acceptable vehicle and used in the preparation.

17. The method for making the compositions according to claim 7, **characterized in that** it comprises the mixing of the active ingredients with pro-apoptotic action with a physiologically acceptable vehicle.

18. The method for making the emulsions according to claim 7, **characterized in that** it includes the step of preparing the oily phase and the aqueous phase in separate containers and hence the combination of the two phases.

19. The method for making the emulsions according to claim 7, **characterized in that** the oily phase is obtained by mixing its components by constant stirring in a mixing container separated from the mixing container containing the components of the aqueous phase, for a time depending on the size of the mixing container used and varying from a few minutes to hours, being the oily phase heated at a temperature of about 40° up to 90 °C.

20. The method for making the emulsions according to claim 7, **characterized in that** the aqueous phase is obtained by dissolution of solid and liquid components in water under gentle stirring, for a time depending on the size of the mixing container used (varying from a few minutes to hours, being the aqueous phase heated at the same melting temperature of the oily phase, before mixing the two phases, in order to obtain the emulsion.

21. The method for making the emulsions according to claim 7, **characterized in that** the oily phase is mixed together with the aqueous one under constant stirring until it becomes homogeneous.

22. The method for making the emulsions according to claim 7, **characterized in that** the mixing of the oily phase and the aqueous phase takes place in the same container or tank in which the aqueous or oily phase were originally mixed.

23. The method for making the emulsions according to claim 7, **characterized in that** the resulting mixture is cooled by circulation of cold water in the heating jacket of the container in which the preparation is carried out until the mixture reaches the desired temperature.

24. The method for making the emulsions according to claim 7, **characterized in that** one or more components is added to the mixture during or after the cooling phase under continuous slow mixing, at the end of the process the mixture being stored in a storage tank and then packaged in the final containers.

25. The method for making the compositions in the gel form according to claim 7, **characterized in that** it provides for an aqueous phase subsequently gelled, the aqueous phase being obtained by dissolution of solid and liquid components in water under stirring, for a time which depends on the intensity of the stirring itself and the size of the mixing container used, and being gelled with a gelling agent that can be added to the aqueous phase during the stirring process or at the end of the same, also providing for the control and correction of the final pH of the product.

26. The method for making the compositions in the form of sprays, shampoos, solutions and lotions according to claim 7, **characterized in that** it provides for an aqueous phase obtained by dissolution of the solid and liquid components in water under stirring, for a time which depends on the intensity of the stirring itself and on the size of the mixing container used, the aqueous phase being adequately perfumed, coloured and/or thickened with perfumes, dyes or thickeners added during the stirring process or at the end of it, also providing for the control and correction of the final pH of the product.

27. A combination of natural active ingredients consisting of compositions **characterized in that** they comprise resin extracts of *Boswellia Serrata* or its pure active ingredients of the family of Boswellic acids and phytosterols or phytostanols or derivatives thereof for use in promoting apoptosis in damaged or suffering skin tissues.
